# NEUE EUROPÄISCHE PATENTSCHRIFT

(11) **EP 0 158 280 B2**
(45) Veröffentlichungstag und Bekanntmachung des Hinweises auf die Entscheidung über den Einspruch: **07.04.1993**
(45) Hinweis auf die Patenterteilung: 14.01.1987
(21) Anmeldenummer: 85104064.2
(22) Anmeldetag: 03.04.1985
(51) Int. Cl.: C07C 7/11, C07C 11/04

(54) **Verfahren zur Entfernung von Acetylen aus einem C2-Strom**
Process for removing acetylene from a C2 stream
Procédé pour l'élimination d'acétylène d'un courant en C2

(30) Priorität: 13.04.1984 DE 3413898
(43) Veröffentlichungstag der Anmeldung: 16.10.1985
(73) Patentinhaber: EC ERDÖLCHEMIE GMBH, D-50769 Köln (DE)
(72) Erfinder: Ruch, Siegfied, Dr., D-4047 Dormagen 1 (DE); Hagen, Karl Heinz, D-5090 Leverkusen 3 (DE); Hambrock, Klaus-Peter, D-5024 Pulheim (DE)
(74) Vertreter: Zobel, Manfred, Dr.

(56) Entgegenhaltungen:
- DE-A- 1 944 505
- DE-B- 2 410 713
- DE-C- 953 700
- GB-A- 636 279
- US-A- 2 250 925
- US-A- 2 806 552
- US-A- 2 942 042

## Beschreibung

Die vorliegende Erfindung betrifft ein Verfahren zur Entfernung von Acetylen aus rohen C₂-Strömen durch eine Wäsche mit einem Absorptionsmittel in einer Absorptionskolonne sowie durch weitere Einspeisung eines flüssigen, weitgehend Acetylen-freien C₂-Stroms in diese Absorptionskolonne.

Bei der Herstellung von Ethylen durch thermische Spaltung von Kohlenwasserstoffen, wie Ethan, Propan, Flüssiggas, Naphtha oder Gasöl wird auch Acetylen in kleiner Menge mitgebildet. Die Zerlegung des bei einer solchen thermischen Spaltung erhaltenen Gasgemisches ergibt Fraktionen von Kohlenwasserstoffgruppen mit gleicher Kohlenstoffzahl, so daß das Acetylen gemeinsam mit Ethan und Ethylen in die C₂-Fraktion gelangt, die zur Gewinnung von reinem Ethylen weiter aufgearbeitet werden muß.

Ethylen findet in großem Maßstab Verwendung zu Ethylierungen, Oligomerisierungen und vor allem zur Polymerisation. Hierbei werden erhebliche Anforderungen an die Reinheit des Ethylens gestellt; beispielsweise darf der Acetylengehalt des Ethylens für die Polymerisation nicht mehr als 5 ppm betragen.

Zur Erreichung dieser Reinheit können zwei Wege beschritten werden. Der eine Weg ist die selektive Hydrierung des Acetylens an verschiedenen Stellen der Aufarbeitung der C₂-Fraktion; der zweite Weg ist die Absorption des Acetylens mit Hilfe eines selektiv wirkenden Absorptionsmittels, mit welchem es vom verbleibenden Ethylen/Ethan-Gemisch abgetrennt wird. Die Selektivhydrierung des Acetylens erfordert zwar einen geringeren apparativen Aufwand, sie hat jedoch den Nachteil, daß hierbei auch ein Teil des Ethylens durch Überhydrierung als Ethan verlorengeht. Die absorptive Abtrennung des Acetylens hat bei höherem apparativen Aufwand dagegen den Vorteil, daß kein Ethylen zu Ethan umgewandelt wird und daß das Acetylen als hochwertiger Rohstoff in reiner Form gewonnen werden kann.

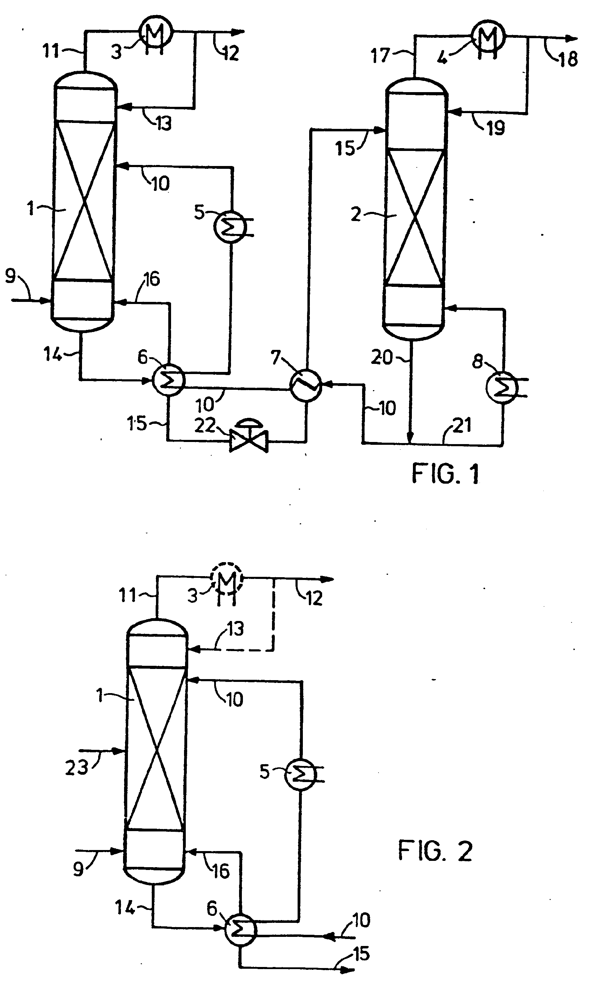

Die absorptive Entfernung von Acetylen aus einem rohen C₂-Strom ist bekannt und sei anhand der beigefügten Figur 1 erläutert:

Das Absorptionsverfahren umfaßt als hauptsächliche Apparate eine Absorptionskolonne (1), eine Regenerierkolonne (2)fürdas mit Acetylen beladeneAbsorptionsmittel, Kondensatoren (3) und (4) bzw. Kühler (5) sowie Wärmeaustauscher (6) und (7) für den Wärmeaustausch zwischen dem regenerierten und dem beladenen Absorptionsmittel, wobei das beladene Regenerationsmittel in einer Entspannungseinrichtung (22) auf einen niedrigeren Druck als in der Absorptionskolonne entspannt wird, und weiterhin einen Umlauferhitzer (8) zur Beheizung der Regenerationskolonne (2). Auf die Darstellung von Pumpen und anderer dem Fachmann geläufiger Beiapparatur wurde in Figur 1 aus Gründen der Übersichtlichkeit verzichtet.

Die Stoffströme in Figur 1 sind der rohe, Acetylenhaltige C₂-Strom (9), der gasförmig unten in die Absorptionskolonne eingespeist wird und der im Gegenstrom durch herabfließendes Absorptionsmittel (10) vom Acetylen befreit wird; das Kopfprodukt (11 ) der Absorptionskolonne (1) wird zum Teil als Acetylenfreies Ethylen/Ethan-Gemisch (12) der weiteren Aufarbeitung zugeführt und zum Teil nach Kondensation als Rücklauf (13) wieder auf die Absorptionskolonne (1) gegeben; das im Sumpf der Absorptionskolonne (1) anfallende, mit Acetylen beladene Absorptionsmittel (14) wird im Wärmeaustauscher (6) durch regeneriertes Absorptionsmittel angewärmt, wobei ein Teil der gelösten Gase, vorzugsweise Ethan und Ethylen, ausgast und als gasförmiger Strom (16) in den unteren Teil der Absorptionskolonne (1) zurückgeleitet wird; das nunmehr mit Acetylen angereicherte Absorptionsmittel (15) gelangt nach Entspannung in der Entspannungsapparatur (22) und erneuten Wärmeaustausch im Wärmeaustauscher (7) in die Regenerierungskolonne (2); das Acetylen-reiche Kopfprodukt (17) der Regenerierungskolonne (2) wird in (4) abgekühlt, der Strom (18) zur Aufarbeitung auf Acetylen entnommen und das auskondensierte Absorptionsmittel (19) auf die Regenerierungskolonne (2) zurückgeführt; das im Sumpf von (2) anfallende regenerierte Absorptionsmittel (20) wird zum Teil als Strom (21 ) über den Umlauferhitzer (8) nach (2) zurückgeführt, zum Teil als Strom (10) über die Wärmeaustauscher (7) und (6) nach (1) zurückgeführt, wobei in (5) die erforderliche Temperatur eingestellt wird.

Das geschilderte Absorptionsverfahren hat einen hohen technischen Stand erreicht. Dennoch haftet ihm in der Notwendigkeit des C₂-Rückflusses (13) ein beträchtlicher Nachteil an. Dieser Rückfluß bringt zum einen eine zusätzliche Belastung der Absorptionskolonne mit C₂-Stoffen. Weiterhin kommt es an der Übergangsstelle zwischen dem Rückflußteil und der Einspeisestelle des Absorptionsmittels in der Absorptionskolonne zu instabilen Betriebsverhältnissen, die im höheren Lastbereich der Absorptionskolonne zum Stauen führen. Um eine sichere Betriebsweise zu gewährleisten, muß daher ein genügend großer Abstand gegenüber diesem Staupunkt eingehalten werden, was in einer bestehenden Anlage zu Kapazitätsbeschränkungen und bei einer Neuanlage zur Notwendigkeit von größeren Apparaturen führt. Weiterhin befindet sich das für den Rückfluß (13) eingesetzte Kondensat im Temperatur-Druckgleichgewicht, was bei nur geringen Schwankungen von Druck oder Temperatur zum Ausgasen der Flüssigkeit an der Saugseite der Förderpumpe für den Rückfluß führt; ein solches Ausgasen führt zum Kavitieren der Pumpe. Eine solche Kavitation führt nicht nur zu einem beschleunigten Verschleiß der Pumpe, sondern läßt auch den Rückfluß auf die Absorptionskolonne ausfallen. Durch den Austall des Rückflusses steigt die Temperatur der Absorptionskolonne sprunghaft an, so daß Acetylen in das Kopfprodukt durchbricht. In solchen Fällen muß dann die gesamte durch Acetylen kontaminierte Ethylenproduktion wegen Überschreitung der Acetylen-Spezifikationsgrenze als wertloses Material verbrannt werden.

Es ist bereits versucht worden, die geschilderten Unregelmäßigkeiten unter Kontrolle zu bekommen, beispielsweise durch Vorsättigen des Absorptionsmittels mit Ethylen, durch geänderte Temperaturführung der Absorptionskolonne oder durch die Zugabe von Antischaummitteln. Die genannten Lösungsversuche waren jedoch sämtlich erfolglos.

Überraschend wurde gefunden, daß durch die Einspeisung eines flüssigen und weitgehend Acetylen-freien C₂-Stroms in die Absorptionskolonne die Menge des Rückflusses am Kopf der Absorptionskolonne beträchtlich vermindert und sogar völlig überflüssig gemacht werden kann, so daß die genannten Nachteile vermindert werden oder völlig aufgehoben werden können.

Die Erfindung betrifft daher ein Verfahren zur Entfernung von Acetylen aus einem rohen C₂-Strom durch eine Wäsche mit einem Absorptionsmittel, das ein selektives Lösevermögen für Acetylen gegenüber Ethylen und Ethan aufweist, in einer Absorptionskolonne, das dadurch gekennzeichnet ist, daß ein flüssiger, weitgehend Acetylen-freier C₂-Strom zwischen den Einspeisestellen für den rohen C₂-Strom und für das Absorptionsmittel in die Absorptionskolonne eingespeist wird.

Als flüssiger weitgehend Acetylen-freier C₂-Strom kommt flüssiges Ethan, flüssiges Ethylen oder ein flüssiges Ethan/Ethylen-Gemisch in Frage. Die genannten Stoffe können bis zu 2 % Acetylen, bevorzugt bis zu 1 % Acetylen, besonders bevorzugt bis zu 0,1 % Acetylen enthalten. Solche C₂-Ströme stehen an vielen Stellen in einer Anlage zur Herstellung von Ethylen zur Verfügung, beispielsweise das Sumpfprodukt der Methansäule, das Heizkondensat der Ethylensäule, das Kondensat des Kopfproduktes der Absorptionskolonne sowie das bei der Ethylen-Gewinnung erhältliche reine Ethylen oder weitgehend vom Ethylen befreite Ethan. Wegen der besonders guten Verfügbarkeit wird ein Ethan/Ethylen-Kondensat, beispielsweise das der Absorptionskolonne, oder reines Ethylen als erfindungsgemäßer C₂-Strom eingesetzt. Der Einsatz von reinem Ethylen ist hierbei besonders bevorzugt, weil mit reinem Ethylen keine im Sinne der Ethylen-Gewinnung als Ballaststoffe anzusehenden fremden Stoffe eingeschleust werden.

Solche erfindungsgemäß einzusetzenden flüssigen C₂-Strö -me liegen mit einer Temperatur von - 100° C bis -30° C und bei einem Druck vor, der mindestens dem zur Verflüssigung einer solchen C₂-Stromes bei der genannten Temperatur erforderlichen Druck entspricht. In bevorzugter Weise wird ein C₂-Strom eingesetzt, der den Temperatur und Druckbedingungen der Absorptionskolonne entspricht. Die Absorptionskolonne wird in einem Druckbereich von 4 - 20 bar, bevorzugt 5 - 15 bar, und der zugehörigen Sattdampftemperatur des zu reinigenden rohen C₂-Stromes betrieben; dieser Temperaturbereich bewegt sich etwa von -74° C bis etwa -30°C. Unter den genannten Gegebenheiten ist es wiederum bevorzugt, den erfindungsgemäß einzuspeisenden flüssigen C₂-Strom von einem höheren Druckniveau zu entnehmen, als es in der Absorptionskolonne herrscht; durch diese Maßnahme kann auf eine Förderpumpe verzichtet werden, wodurch eventuelle Kavitationserscheinungen an deren Saugseite und die oben diskutierten Störungen vermieden werden.

Der erfindungsgemäß einzuspeisende flüssige C₂-Strom wird in einer Menge von 1 - 4 %, bevorzugt 1,3 - 3,3 %, besonders bevorzugt 1,5 - 2,5 %, bezogen auf die Menge des rohen C₂-Stroms, in die Absorptionskolonne eingespeist. Für den Fall, daß die Absorptionskolonne und damit auch der einzuspeisende C₂-Strom eine tiefere Temperatur im angegebenen Bereich der Sattdampftemperaturen haben, kann die einzuspeisende Menge des flüssigen C₂-Strom im unteren Teil des angegebenen Mengenbereichs sein und umgekehrt.

Der flüssige weitgehend Acetylen-freie C₂-Strom wird erfindungsgemäß zwischen den Einspeisestellen für den rohen C₂-Strom und für das Absorptionsmittel in die Absorptionskolonne eingespeist. Hierbei wird von einer Absorptionskolonne mit insgesamt 5 - 30 Böden, bevorzugt 5-20 Böden, ausgegangen. Der rohe C₂-Strom wird hierbei unterhalb des ersten Bodens oder auf den 1. - 5. Boden, bevorzugt unterhalb des 1. Bodens, eingespeist. Auch das aus dem Sumpfablauf (14) im Wärmeaustauscher (6) ausgegaste Rückgas (16) wird unabhängig vom rohen C₂-Strom unterhalb des 1. Bodens oder auf den 1. - 5. Boden, bevorzugt unterhalb des 1. Bodens eingespeist.

Das Absorptionsmittel wird auf den 3. - 6. Boden vom oberen Ende der Kolonne eingespeist.

Der erfindungsgemäße flüssige weitgehend Acetylen-freie C₂-Strom wird nun an einer Stelle vom 2. Boden der Absorptionskolonne bis unterhalb der Einspeisestelle des Absorptionsmittels eingespeist, wobei in Übereinstimmung mit dem oben Gesagten der rohe C₂-Strom noch unterhalb des erfindungsgemäßen flüssigen C₂-Stromes eingespeist wird. In bevorzugter Weise wird der erfindungsgemäße flüssige C₂-Strom mindestens drei Böden unterhalb der Einspeisestelle für das Absorptionsmittel eingepeist.

Im erfindungsgemäßen Verfahren kann wie bei herkömmlichen Verfahren mit einem Rücklauf auf den Kopf der Absorptionskolonne gearbeitet werden. In bevorzugter Weise wird jedoch dieser Rücklauf gegenüber den bisher als notwendig angesehenen Mengen vermindert. Als Verminderung des Rücklaufs kommt hierbei mindestens die Menge in Betracht, die dem erfindungsgemäß einzuspeisenden flüssigen weitgehend Acetylen-freien C₂-Stroms entspricht. Wenn beispielsweise zur Erzielung eines bezüglich des Acetylengehalts spezifikationsgerechten Ethylens ein Rücklauf auf die Absorptionskolonne von 10 - 15 % des gesamten C₂-Durchsatzes für erforderlich gehalten wurde und die Einspeisung des flüssigen weitgehend Acetylen-freien C₂-Stroms mit den oben angeführten Mengen von 1 - 4 % des gesamten C₂-Durchsatzes angesetzt wird, kann der Rücklauf auf den Kopf der Absorptionskolonne im Rahmen des erfindungsgemäßen Verfahrens auf Werte von 6 - 14 % des gesamten C₂-Durchsatzes gesenkt werden. In weiterhin bevorzugter Verfahrensweise wird der Rücklauf jedoch um eine größere Menge verkleinert als dem erfindungsgemäß eingespeisten flüssigen C₂-Strom entspricht, beispielsweise eine Verminderung um das 1 - 10-fache des erfindungsgemäß eingespeisten flüssigen C₂-Stroms, wobei die Obergrenze dieses Bereiches (Verminderung um das 10-fache) beispielsweise für den Fall steht, daß bei der herkömmlichen Verfahrensvariante ein Rücklauf in Höhe von 10 % (15 %) der gesamten C₂-Einspeisung für erforderlich gehalten wurde und die erfindungsgemäße Einspeisung des flüssigen C₂-Stroms in Höhe von 1 % (1,5 %) des gesamten C₂-Durchsatzes angesetzt wurde. In diesem Extremfall wird also auf den bisher für erforderlich gehaltenen Rücklauf vollständig verzichtet. Es hat sich nun überraschend gezeigt, daß mit dieser Variante, also dem vollständigen Verzicht des bisher für erforderlich gegehaltenen Rücklauf, ausgezeichnete Ergebnisse erhalten werden, so daß der völlige Verzicht auf den Rücklauf eine besonders bevorzugte Variante des erfindungsgemäßen Verfahrens darstellt.

Als Absorptionsmittel kommen alle in Frage, die ein selektives Lösevermögen für Acetylen gegenüber Ethylen und Ethan aufweisen, z.B. N-Alkyl-pyrrolidone, beispielsweise N-Methyl-pyrrolidon-(2), Dialkylformamide, beispielsweise Dimethytformamid oder Diethylformamid, und niederealiphatische Ketone, beispielsweise Aceton oder Methyl-ethylketon. Für den Fall, daß diese selektiv lösenden Absorptionsmittel einen Schmelzpunkt haben, der oberhalb der angestrebten Arbeitstemperatur der Absorptionskolonne liegt, kann das Absorptionsmittel im Gemisch mit tiefsiedenden niederen aliphatischen Alkoholen eingesetzt werden, beispielsweise im Gemisch mit Methanol, Ethanol, n-Propanol i-Propanol n-Butanol oder sek-Butanol. Der Gehalt an dem genannten tiefsiedenden Alkohol im Gemisch mit dem selektiv lösenden Absorptionsmittel kann in bekannter Weise 5 - 30 Gew.-% betragen.

Das erfindungsgemäße Verfahren weist folgende Vorteile auf, die bisher nicht für möglich gehalten wurden:
1. Der Rücklauf auf die Absorptionskolonne kann vermindert werden oder ganz entfallen.
2. Im Zusammenhang mit 1. kann der Energiebedarf für die Acetylenabtrennung, insbesondere bei den bevorzugten Verfahrensvarianten, reduziert werden, da die erfindungsgemäße Einspeisung des flüssigen C₂-Stromes beträchtlich kleiner ist als der bisher für erforderlich gehaltene Rücklauf.
3. Durch die Verminderung oder das gänzliche Entfallen des Rücklaufs wird das sonst beobachtete Stauen der Säule im höheren Lastbereich vermindert oder entfällt gänzlich; hierdurch wird eine sichere Betriebsweise gewährleistet.
4. Im Zusammenhang mit 3. kann die Kapazität der Absorptionskolonne bei gleicher Trennleistung um mindestens 6 %, in vielen Fällen um etwa 16 % der ursprünglichen Nennleistung erhöht werden.
5. Bei Neuanlagen kann auf die oberen Rückflußböden, den Kondensator für das Kopfprodukt sowie den Rückflußbehälter einschließlich der zugehörigen Pumpen verzichtet werden, wodurch die Investitionskosten gesenkt werden.
6. Wenn die erfindungsgemäße C₂-Einspeise- flüssigkeit aus einer Verfahrensstufe mit höherem Druckniveau als dem der Absorptionskolonne entnommen wird, kann auf eine Pumpenförderung verzichtet werden, wodurch die bekannten Nachteile, beispielsweise die Kavitation auf der Pumpensaugseite, entfallen. Auch hierdurch wird die Zuverlässigkeit des Dauerbetriebes nennenswert erhöht; Produktabwertungen, beispielsweise durch Verbrennen nicht spezifikationsgerechten Ethylens im Störfalle, wobei lediglich der Heizwert berechnet werden kann, werden vermieden.
7. Der Acetylen-freie Kopfstrom derAbsorptionskolonne fällt bei Verminderung oder bei völligem Entfallen des Rücklaufes mit höherer Temperatur an und bringt für die nachfolgende Ethylen/Ethan-Trennung einen zusätzlichen Energievorteil; so kann damit gerechnet werden, daß der Kopfstrom statt bisher mit etwa -48° C nach dem erfindungsgemäßen Verfahren mit etwa -30°C anfällt und nicht mehr kondensiert zu werden braucht, da das Ethylen/Ethan-Gemisch im allgemeinen gasförmig in die Ethylen/Ethan-Trennung eingefahren wird.

Die allgemeine Ausführung des erfindungsgemäßen Verfahrens sei anhand von Figur dargestellt. Figur enthält nur die Absorptionskolonne (1), an die sich die Regenerierungskolonne (2), wie sie in Figur 1 gezeigt wurde, unverändert anschließt. Soweit die Apparate und Stoffströme mit den in Figur 1 gezeigten übereinstimmen, tragen sie die gleiche Bezifferung. Als (23) ist in Figur 2 zusätzlich zu Figur 1 der erfindungsgemäß einzuspeisende flüssige und weitgehend Acetylen-freie C₂-Strom eingezeichnet. Der Rücklauf (13) auf (1) sowie der Wärmetauscher (3) ist in Figur 2 gestrichelt gezeichnet, da er, entsprechend der vorangegangenen Beschreibung, wahlweise vermindert werden kann oder ganz entfallen kann.

### Beispiele

### Beispiel 1 (zum Vergleich)

In einer bestehenden thermischen Spaltanlage zur Erzeugung von Ethylen mit einer Auslegungskapazität von 366 400 jato Ethylen erfolgt die Acetylenabtrennung mit N-Methyl-pyrrolidon (NMP)/Methanol-Gemisch im Gewichtsverhältnis von 85/15. Die Absorptionskolonne enthält 24 Glockenböden und ist in 2 Säulenabschnitte unterteilt. Über die unteren 19 Böden fließt das Absorptionsmittel von oben nach unten im Gegenstrom zu dem aufsteigenden rohen C₂-Strom, während über die oberen 5 Böden ein Acetylen-freies C₂-Kopfkondensat als Rücklauf gefahren wird. Bei Überschreitung des Auslegungswertes von 55 t/h Einsatz an rohem C₂-Strom um mehr als 2 % kam es sofort zum Stauen der Absorptionskolonne und damit zu Produktionsschwierigkeiten und Produktverlust, weil nicht spezifikationsgerechtes Ethylen lediglich als Heizgas verwendet werden konnte. Der Rücklauf betrug hierbei 6,6 t/h.

### Beispiel 2 (erfindungsgemäß)

In die gleiche Absorptionskolonne wie im Beispiel 1 wurde das Absorptionsmittel NMP/Methanol wie bisheraufden 19. Boden gegeben. Aufden 12. Boden wurde ein flüssiger Strom von reinem Ethylen in einer Menge von 1,2 t/h gegeben. Der Rücklauf konnte völlig entfallen. Der Einsatz an rohem C₂-Strom konnte bis auf 64 t/h erhöht werden, ohne daß es zum Stauen der Säule kam. Die Durchsatzsteigerung entspricht einer Ethylenproduktion von 426 400 jato.

## Patentansprüche

1. Verfahren zur Entfernung von Acetylen aus einem rohen C₂-Strom durch eine Wäsche mit einem Absorptionsmittel, das ein selektives Lösevermögen für Acetylen gegenüber Ethylen und Ethan aufweist, in einer Absorptionskolonne, dadurch gekennzeichnet, daß ein flüssiger weitgehend Acetylenfreier C₂-Strom zwischen den Einspeisestellen für den rohen C₂-Strom und für das Absorptionsmittel in die Absorptionskolonne eingespeist wird.

2. Verfahren nach Anspruch 1, dadurch gekennzeichnet, daß als einzuspeisender C₂-Strom ein Ethylen-, Ethanoder Ethylen/Ethan-Strom eingesetzt wird.

3. Verfahren nach Anspruch 1, dadurch gekennzeichnet, daß reines Ethylen eingesetzt wird.

4. Verfahren nach Ansprüchen 1 bis 3, dadurch gekennzeichnet, daß der flüssige C₂-Strom 1 - 4 Gew.-% des rohen C₂-Stroms beträgt.

5. Verfahren nach Ansprüchen 1 bis 3, dadurch gekennzeichnet, daß der flüssige C₂-Strom 1,3-3,3 Gew.-% des rohen C₂-Stroms beträgt.

6. Verfahren nach Ansprüchen 1 bis 3, dadurch gekennzeichnet, daß der flüssige C₂-Strom 1,5-2,5 Gew.-% des rohen C₂-Stroms beträgt.

7. Verfahren nach Ansprüchen 1 bis 6, dadurch gekennzeichnet, daß der Rücklauf des Kopfproduktes der Absorptionskolonne auf die Absorptionskolonne um mindestens die Menge des eingespeisten C₂-Stromes vermindert wird.

8. Verfahren nach Ansprüchen 1 bis 7, dadurch gekennzeichnet, daß ohne Rückfluß gearbeitet wird.

9. Verfahren nach Ansprüchen 1 bis 8, dadurch gekennzeichnet, daß ein C₂-Strom eingespeist wird, der im weiteren Aufarbeitungsprozeß des Kopfproduktes der Absorptionskolonne bei einem höheren Druck vorliegt als dem Druck in der Absorptionskolonne.

## Claims

1. Process for removing acetylene from a crude C₂- stream by scrubbing with an absorbent which has a selective solvency for acetylene as opposed to ethylene and ethane, in an absorption column, characterised in that a liquid, substantially acetylene-free C₂-stream is fed into the absorption column between the feed points for the crude C₂- stream and for the absorbent.

2. Process according to Claim 1, characterised in that an ethylene, ethane or ethylene/ethane stream is used as the C₂-stream to be fed in.

3. Process according to Claim 1, characterised in that pure ethylene is used.

4. Process according to Claims 1 to 3, characterised in that the liquid C₂-stream is 1-4% by weight of the crude C₂-stream.

5. Process according to Claims 1 to 3, characterised in that the liquid C₂-stream is 1.3-3.3% by weight of the crude C₂-stream.

6. Process according to Claims 1 to 3, characterised in that the liquid C₂-stream is 1.5-2.5% by weight of the crude C₂-stream.

7. Process according to Claims 1 to 6, characterised in that the reflux of the top product of the absorption column onto the absorption column is reduced by at least the amount of the C₂-stream fed in.

8. Process according to Claims 1 to 7, characterised in that it is carried out without reflux.

9. Process according to Claims 1 to 8, characterised in that a C₂-stream is fed in which, in the subsequent working-up process of the top product of the absorption column, is under a higher pressure than the pressure in the absorption column.

## Revendications

1. Procédé d'élimination de l'acétylène à partir d'un courant brut de C₂ par un lavage avec un agent d'absorption qui a un pouvoir dissolvant sélectif à l'égard de l'acétylène par rapport à l'éthylène et à l'éthane, dans une colonne d'absorption, caractérisé en ce qu'on alimente un courant de C2liquide, largement exempt d'acétylène, entre les endroits d'alimentation pour le courant de C₂ brut et pour l'agent d'absorption dans la colonne d'absorption.

2. Procédé selon la revendication 1, caractérisé en ce qu'on utilise comme courant de C₂ d'alimentation un courant d'éthylène, d'éthane ou d'éthylè- ne/éthane.

3. Procédé selon la revendication 1, caractérisé en ce qu'on utilise de l'éthylène pur.

4. Procédé selon les revendications 1 à 3, caractérisé en ce que le courant de C₂ liquide s'élève à 1 à 4% en poids du courant de C₂ brut.

5. Procédé selon les revendications 1 à 3, caractérisé en ce que le courant de C₂ liquide s'élève à 1,3 à 3,3% en poids du courant de C₂ brut.

6. Procédé selon les revendications 1 à 3, caractérisé en ce que le courant de C₂ liquide s'élève à 1,5 à 2,5% en poids du courant de C₂ brut.

7. Procédé selon les revendications 1 à 6, caractérisé en ce que le reflux du produit de tête de la colonne d'absorption renvoyé à la colonne d'absorption est diminué d'au moins la quantité du courant de C₂ alimenté.

8. Procédé selon les revendications 1 à 7, caractérisé en ce qu'on opère sans reflux.

9. Procédé selon les revendications 1 à 8, caractérisé en ce qu'on alimente un courant de C₂ qui, au cours du processus ultérieur de traitement du produit de tête de la colonne d'absorption, se présente sous une pression supérieure à la pression régnant dans la colonne d'absorption.
